# EUROPEAN PATENT APPLICATION

(11) **EP 2 388 594 A1**
(43) Date of publication of application: **23.11.2011**
(21) Application number: 10162966.5
(22) Date of filing: 17.05.2010
(51) Int. Cl.: G01N 33/68

(54) **GDF-15 based means and methods for survival and recovery prediction in acute inflammation**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 68259 Mannheim (DE); Zdunek, Dietmar, 82329 Tutzing (DE)
(74) Representative: Dick, Alexander

(57) **Abstract**

The present invention relates to the field of diagnostic measures. Specifically, it relates to a method for diagnosing whether a subject suffering from an acute inflammation and, preferably, systemic inflammtory response syndrome (SIRS), is at increased risk for mortality, said method comprises determining the amount of the biomarker GDF-15 in a sample of said subject and comparing said amount to a reference whereby an increased risk for mortality is to be diagnosed. Moreover, the present invention relates to a method for monitoring the development of acute inflammation in a subject suffering therefrom comprising determining the amount of the biomarker GDF-15 in a first and a second sample of said subject wherein said first sample has been obtained prior to said second sample and comparing the amount of GDF-15 in said first and said second sample wherein an increase in the GDF-15 amount is indicative for a diagnosis of progression of acute inflammation whereas a decrease in the amount of GDF-15 is indicative for the diagnosis of an amelioration of said acute inflammation. Further encompassed are diagnostic devices and kits for carrying out the aforementioned methods.

## Description

The present invention relates to the field of diagnostic measures. Specifically, it relates to a method for diagnosing whether a subject suffering from an acute inflammation and, preferably, systemic inflammatory response syndrome (SIRS), is at increased risk for mortality, said method comprises determining the amount of the biomarker GDF-15 in a sample of said subject and comparing said amount to a reference whereby an increased risk for mortality is to be diagnosed. Moreover, the present invention relates to a method for monitoring the development of acute inflammation in a subject suffering therefrom comprising determining the amount of the biomarker GDF-15 in a first and a second sample of said subject wherein said first sample has been obtained prior to said second sample and comparing the amount of GDF-15 in said first and said second sample wherein an increase in the GDF-15 amount is indicative for a diagnosis of progression of acute inflammation whereas a decrease in the amount of GDF-15 is indicative for the diagnosis of an amelioration of said acute inflammation. Further encompassed are diagnostic devices and kits for carrying out the aforementioned methods.

Fever is a frequent event and caused by infections, microbial toxins, mediators of inflammation and immune reactions. It is associated with the release of pyrogenic cytokines such as Il 1, Il 6, TNF and interferons. These cytokines are responsible for the release of prostaglandin E 2 (PGE 2) in peripheral tissues.( Dinarello C.A., Porat R., in Principles of Internal Medicine 17. Ed p 117 ff. ). Most fevers are associated with self limited infections, such as common viral diseases and are thus harmless. Thus, in most cases fever can be treated with antipyretics in order to achieve relief of symptoms. Antipyretics might however be contraindicated in patients with bacterial infections as they may mask inadequately treated bacterial infections. Specifically bacterial and fungal infections may become systemic and might progress to systemic inflammatory response syndrome (SIRS) which is characterized by hyper- or hypothermia, tachypnoe, tachycardia and leucocytosis and leucopenia.

SIRS associated with proven or suspected infection is called Sepsis. Sepsis can proceed to severe sepsis, septic shock, refractory septic shock or multi-organ dysfunction syndrome (MODS). The latter conditions are associated with organ dysfunction, e.g. the cardiovascular, the renal, the respiratory, the cerebral or the hematologic system. Attempts to improve survival or systemic infections, specifically sepsis have failed in the past. The major reason for treatment failure was the clinically apparent development of organ dysfunction which could not be reversed by treatment. Thus there is an urgent need to identify individuals at risk of complications early to provide them with timely treatment (e.g. antibiotics). (Munford in Harrison Principles of Internal Medicine, 17, p.1695 ff). In fact, time elapsed to administration of appropriate treatment is the primary determinant of mortality in patients with sepsis (Gaieski 2010, Crit Care Med 38 1045-1053).

Current methods to assess prognosis of a patient, preferably in the intensive care unit (ICU), includes the APACHE II score as well as the SOFA Score. The APACHE II Score includes the assessment of temperature, mean blood pressure, heart rate, respiratory rate, arterial pH, oxygenation, serum sodium, serum potassium, hematocrit and white blood cell count.. In addition age, chronic health status and the Glasgow Coma score is considered..Various studies indicate that the APACHE II score is useful in predicting survival, survival rates have been shown to be higher in postoperative cases if compared with nonoperative cases at the same APACHE II score level. The Apache II scoring system is not restricted to the ICU but can also be performed in the emergency room, this system is however time consuming and as is cannot differentiate between different organ failures.(Kress and Hall in Harrison Principles of Internal Medicine, 17, p. 1673 ff ).

An alternative to the APACHE II scoring system is the SOFA Score. The SOFA Score (Sepsis -related organ Failure Assessment) includes the determination of oxygenation (PaO₂/Fi=2 (mmHg), platelet counts, bilirubin. Blood pressure, Glasgow coma scale as well as kidney function (creatinin and urine volume/day); see Vincent 1996, Intensive Care Med, 22:707-10. The SOFA Scoring system is also associated with prognosis of survival.

However, a disadvantage of both systems are that they are laborious and time consuming and might not pick up early changes/deterioration in organ function, which is of importance in the early recognition of complications and for treatment decisions ( see above) or for the identification of patients at increased risk of complications ( see above).

Growth differentiation factor 15 (GDF 15) also called MIC-1 ( Macrophage inhibitory cytokine 1) is a member of the transforming growth factor beta (TGF beta) family. GDF 15 has been found in macrophages, the placenta, the prostrate and, to a lesser extent, in the liver, the kidney and the brain. GDF 15 has been linked to rheumatoid arthritis, cancer and cardiovascular diseases. In cardiovascular disease GDF 15 has been linked to outcome in acute coronary syndrome as well as in heart failure. Patients suffering from cardiovascular disease are at increased risk to develop infectious complications. Infectious complications may be associated with cardiac involvement (Hunter 2009, Brit J of Anesthesia, 104 (1):3-11). Both troponin and natriuretic peptides have been described in patients with different forms of sepsis and shown to be predictive for outcome in sepsis (Post 2008, Crit. Care Med, 36:3030-3037, Piracchio 2008, Crit Care Med, 36:2542-2546, Mc Lean 2008, Critical Care, 12:1-9).

The technical problem underlying the present invention can be seen as the provision of means and methods for reliably and efficiently predicting adverse outcomes such as mortality in subjects suffering from acute inflammation conditions. The technical problem is solved by the embodiments characterized in the claims and herein below.

Thus, the present invention relates to a method for diagnosing whether a subject suffering from acute inflammation is at increased risk for mortality comprising:
a) determining the amount of the biomarker GDF-15 in a sample of said subject; and
b) comparing said amount to a reference amount whereby an increased risk for mortality is to be diagnosed.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "mortality" as used herein refers to all-cause mortality. Preferably, said mortality is caused by systemic or specific organ failure. Organ systems which may be affected include the cardiac system, the renal system, the liver and metabolic system, the hematopoietic system the respiratory system and/or the central nervous system.

The term "increased risk for mortality" as used herein means that it the subject to be analyzed by the method of the present invention is allocated either into the group of subjects of a population having a normal, i.e. non-elevated, risk for mortality or into a group of subjects having a significantly elevated risk. An elevated risk as referred to in accordance with the present invention means that the risk of mortality within a predetermined predictive window is elevated significantly for a subject with respect to the average risk for mortality in a population of subjects. Preferably, a predictive window to be applied for the method of the present invention is about 30 days. The risk of mortality for a portion of a cohort can be calculated dependent on the threshold amount of a biomarker used to define the said portion by establishing a Kaplan-Meier survival plots.

The term "diagnosing" as used herein means predicting whether the risk for mortality is increased in a subject suffering from acute inflammation, or not. As will be understood by those skilled in the art, such a prediction is usually not intended to be correct for 100% of the subjects to be diagnosed. The term, however, requires that the prediction to be at increased risk for mortality, or not, is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the increased risk for mortality is diagnosed by carrying out the further step of c) diagnosing whether the subject is at increased risk for mortality based on the result of the comparison carried out in step b).

The term "acute imflammation" refers to an acute systemic inflammation response in the subject. Said acute systemic inflammation may result from an infectious cause such as an expanding local infection which cannot be efficiently ameliorated due to an impaired function of the immune system in the subject or may be elicited by a non-infectious cause including trauma, burns, pancreatitis, ischemia and/or hemorrhage. Accordingly, acute systemic inflammation is characterized by a systemically reduced overall immune defense in the subject. The acute systemic inflammation may progress into the even more severe systemic inflammatory response syndrome (SIRS). SIRS as used in accordance with the present invention, preferably, encompasses SIRS as defined on the ACCP/SCCM Consensus Conference Definitions (1992/2003) (see e.g. American College of Chest Physicians/Society of Critical Care Medicine Consensus Conference: definitions for sepsis and organ failure and guidelines for the use of innovative therapies in sepsis. Crit Care Med. 1992 Jun;20(6):864-74)). Preferably, a patient is considered to suffer from SIRS, if the patient displays at least 2 symptoms of the following a) to d), preferably of the following a) to e): a) white blood cell count (WBC) > about 12,000/ µ/L or < about 4000/µ/L, b) body temperature > about 38 0C or < about 36 0C, c) heart rate > about 90 beats/minute, d) a respiratory rate > about 20 breaths/minute or a partial pressure of CO2 of less than about 32 mm Hg, and e) more than 10% immature white blood cells among the counted white blood cells. The white blood cell count is usually determined by automated counting devices. As far as children are concerned, the consensus criteria for diagnosing SIRS in a child are disclosed in Goldstein et al. (Goldstein 2005, Pediatr. Crit Care Med 2005, 6(1),2- 8; see in particular Tables 2 and 3). An asymptomatic child patient in the sense of the present invention is a patient displaying less than 2, preferably less than 1 symptom of the ones described in Goldstein et al. (supra) which is incorporated herein by reference. Furthermore, in cases where SIRS may develop into sepsis if said disorder is accompanied by a proven or suspected microbial etiology, e.g., a systemic infection. An infection in the sense of the present invention preferably is a viral, fungus or bacterial infection, preferably a bacterial infection associated with bacteria selected from E coli, staphylococcus aureus, Klebsiella pneumoniae, Streptococci or Pseudomonas aeroginosa. The infection may as well be an infection by a fungus selected from Candida albicans, Candida tropicalis or Aspergillus fumigatus. An infection is diagnosed on the basis of assays and criteria generally known to the physician. Preferably, the infection is diagnosed on the basis of a bacterial culture assay, e.g. a culture medium inoculated with a sample from the patient, or based on molecular diagnostic methods. A fungus infection may for example be determined based on the generally known test assays such as Septifast. Sepsis may furthermore be determined by the aforementioned criteria for SIRS and at least two of the following criteria in addition: a) presence of leucocytes in a physiologically sterile body fluid; b) peritonitis or perforated viscus; c) pneumonia with focal opacification or petechiae, purpura, or purpura fulminans; d) bacteriemia. Further symptoms or particularly severe complications accompanying SIRS or sepsis are described in standard text books of medicine such as Stadmen or Pschyrembl. More preferably, the acute inflammation as used herein refers to SIRS and/or sepsis.

The term "subject" as used herein relates to animals, preferably mammals, and, more preferably, humans. The subject according to the present invention shall suffer from acute inflammation as described elsewhere herein. Preferably, said subject does not exhibit diseases or disorders selected from impaired kidney function or need of dialysis. Moreover, preferably, the subject does also not exhibit an acute cardiovascular event, in particular, acute coronary syndrome. More preferably, the term "subject" as used herein shall exclude subjects from the method of the invention which suffer from impaired kidney function, preferably, from acute kidney injury.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue-or organ samples are obtained from those cells, tissues or organs which express or produce the peptides referred to herein.

The term "Growth-Differentiation Factor-15" or "GDF-15" relates to a polypeptide being a member of the transforming growth factor beta (TGFβ) cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal antiinflammatory drug-activated gene-1, and prostate-derived factor (Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal propeptide, GDF-15 is secreted as a ~28 kDa dimeric protein (Bauskin 2000, Embo J 19:2212-2220). Amino acid sequences for GDF-15 are disclosed in WO99/06445, WO00/70051, WO2005/113585 Bottner 1999, Gene 237: 105-111, Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

Determining the amount of GDF-15 or any other peptide or polypeptide referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR-analyzers, or chromatography devices. Further, methods include micro-plate ELISA-based methods, fully-automated or robotic immunoassays (available for example on Elecsys™ analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a subject is at increased risk of mortality. Therefore, the reference amount is to be chosen so that either a difference or a similarity in the compared amounts allows identifying those test subjects which belong into the group of subjects having an increased mortality risk, or not. The comparison to be carried out in accordance with the method of the invention also includes the diagnosis of an increased risk for mortality for the subject suffering from acute inflammation or the diagnosis of a normal risk for mortality for the subject suffering from acute inflammation.

Accordingly, the term "reference amount" as used herein refers to an amount which allows assessing whether a subject suffering from acute inflammation has an increased risk for mortality. Accordingly, the reference may e.g. be derived from (i) a subject known to be at increased risk for mortality or (ii) a subject known to be not at increased risk for mortality. Moreover, the reference amount may define a threshold amount or range, whereby dependent on the type of reference a change in the determined amount with respect to the threshold is either indicative for an increased risk for mortality or a normal risk. Alternatively, an essentially identical amount may be either indicative for an increased risk for mortality or a normal risk as well, if a suitable reference amount is used. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can be calculated for a cohort of subjects (i.e. (i) subjects which are known to have an increased risk for mortality or (ii) subjects known to have no increased risk for mortality among the cohort of subjects suffering from acute inflammation) based on the average or mean values for a given biomarker by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/- specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at increased risk for mortality or those which have a normal risk among a cohort of subjects suffering from acute inflammation can be generated, preferably, by establishing a ROC for said cohort as described above and deriving a threshold amount therefrom.

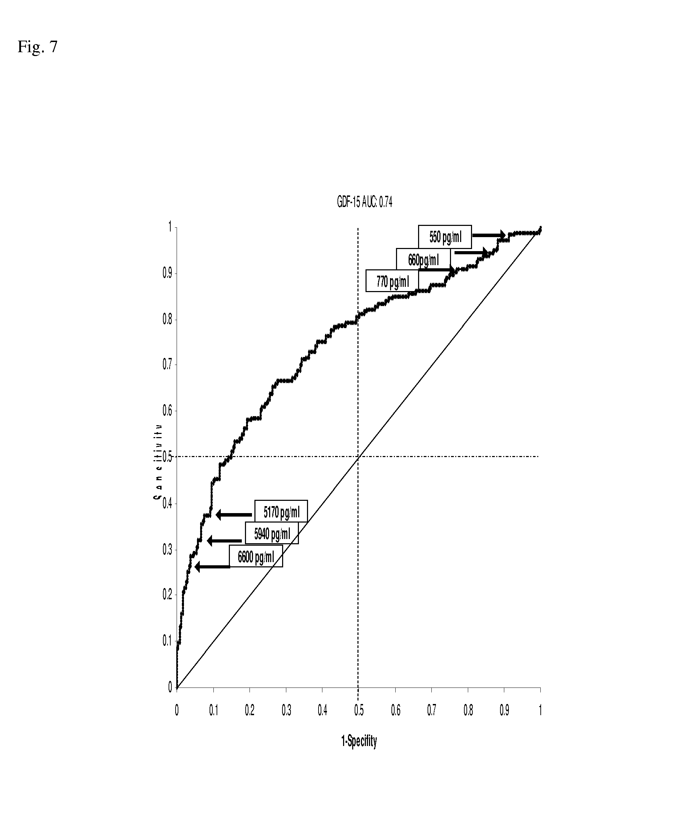

Dependent on a desired sensitivity and specificity for a diagnostic method, the ROC plot allows deriving suitable thresholds. It will be understood that an optimal sensitivity is desired for excluding a subject for being at increased risk (i.e. a rule out) whereas an optimal specificity is envisaged for a subject to be assessed as being at an increased risk (i.e. a rule in). As shown in Figure 7, optimal sensitivity yields threshold amounts to be applicable as reference amounts being indicative for a subject being not at increased risk for mortality are within the range of about 550 pg/ml to about 770 pg/ml, and preferably about 660 pg/ml. Optimal specificity yields threshold amounts to be applicable as reference amounts being indicative for a subject being at increased risk for mortality are within the range of about 5,170 pg/ml to about 6,600 pg/ml, and preferably about 5,940 pg/ml.

About in the context of the present invention means +/- 20%, +/- 10%, +/- 5%, +/- 2 % or +/-1% from the said values. This also takes into account usual deviations caused by measurement techniques and the like.

Preferably, the median values for the biomarker(s) determined in Table 1 may be also used as a basis for establishing thresholds. Preferably, said threshold for GDF-15 for a subject being at increased risk is within the range of the 25^{th} percentile to the 75^{th} percentile, i.e. about 1,870.4 to about 7,161.2 pg/ml, more preferably about 1,980.1 to about 7,623.1 pg/ml or, most preferably, about 5,896.9 to about 22,708.0 pg/ml. More preferably, the median could be used as a threshold and an increased risk is indicated by a value of at least about 3,421.8 pg/ml, 3,652.5 pg/ml or 11,518.0 pg/ml for GDF-15.

In principle, the present invention also relates to a method of diagnosing whether whether a subject suffering from acute inflammation is at increased risk for mortality comprising diagnosing said increased risk for a subject based on a comparison to a reference of the amount of the biomarker GDF-15 in a sample of said subject

Advantageously, it has been found in the studies underlying the present invention that GDF-15 in a body fluid such as blood, plasma or serum can serve as a biomarker allowing to predict an increased risk for mortality in subjects suffering from acute inflammation or even SIRS or sepsis. Thanks to the present invention, it is possible to risk stratify and handle patients suffering from acute inflammation more reliably and in a quality oriented and cost effective manner. Moreover, the time consuming and cumbersome APACHE II and/or SOFA Scoring systems can be avoided or additional information for strengthening the findings made by such scoring systems can be generated by the method of the present invention. The reliable and efficient diagnosis of the severe cases of acute inflammation exhibiting an increased risk for mortality allows for an early therapeutic intervention as well as for applying further precautionary measures to these patients such as a close monitoring of disease progression and intensive care. The method of the present invention can be applied also for the following purposes (i) discrimination of systemic infection from an underlying disease using GDF 15, NT-pro BNP and Troponin T, (ii) complement information from widely used scores (APACHE II and SOFA Score) with regard to cardiac involvement, (iii) in case of indication for systemic infection trigger appropriate treatment and specifically treatment related to the heart disease, (iv) identification of patients with short term poor outcome, and (v) identification of signs of recovery as early as 6 to 24 hours after presentation using GDF-15 or GDF-15 complemented by NT-pro BNP and Troponin T.

It is to be understood that the definitions and explanations of the terms made above and below apply accordingly for all embodiments described in this specification and the accompanying claims.

In a preferred embodiment of the aforementioned method, the amount of the biomarker(s) NT pro-BNP and/or a cardiac troponin is determined in addition to GDF-15.

The term "natriuretic peptide" comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444, by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "cardiac troponin" refers to all troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac troponin refers to troponin T and/or troponin I, and, most preferably, to troponin T. It is to be understood that isoforms of troponins may be determined in the method of the present invention together, i.e. simultaneously or sequentially, or individually, i.e. without determining the other isoform at all. Amino acid sequences for human troponin T and human troponin I are disclosed in Anderson, loc cit and Ferrieres 1998, Clinical Chemistry, 44: 487-493. The term "cardiac troponin" encompasses also variants of the aforementioned specific troponins, i.e., preferably, of tropoinin T or troponin I. Such variants have at least the same essential biological and immunological properties as the specific cardiac troponins. In particular, they share the same essential biological and immunological properties if they are detectable by the same specific assays referred to in this specification, e.g., by ELISA Assays using polyclonal or monoclonal antibodies specifically recognizing the said cardiac troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific troponin. Variants may be allelic variants or any other species specific homologs, paralogs, or orthologs. Moreover, the variants referred to herein include fragments of the specific cardiac troponins or the aforementioned types of variants as long as these fragments have the essential immunological and biological properties as referred to above. Such fragments may be, e.g., degradation products of the troponins. Further included are variants which differ due to posttranslational modifications such as phosphorylation or myristylation.

It will be understood that in the preferred embodiment, also, said reference is, preferably, derived from a subject or a group of subjects suffering from acute inflammation known to be at increased risk for mortality. More preferably, an essentially identical or increased amount for the biomarkers in the sample compared to the reference is indicative for a subject being at increased risk for mortality and wherein a decreased amount for the biomarkers in the sample compared to the reference is indicative for a subject being not at increased risk for mortality. Alternatively, but nevertheless preferred, the said reference is derived from a subject or group of subjects suffering from acute inflammation known to be not at increased risk for mortality. More preferably in such a case an essentially identical or decreased amount for the biomarkers in a sample compared to the reference is indicative for a subject being not at increased risk for mortality and wherein an increased amount for the biomarkers in the sample compared to the reference is indicative for a subject being at increased risk for mortality. The terms "increased" or "decreased" as used herein refer to statistically significant changes, i.e. a statistically significant increase or decrease, respectively. Essentially identical is an amount which does not differ statistically significant from the reference amount. Suitable tests for determining whether changes are statistically significant, or not, are well known in the art. Suitable references for the aforementioned biomarkers which may serve as thresholds can be calculated as described for GDF-15, above. The considerations made for median based thresholds or ROC derived thresholds apply mutatis mutandis for the biomarkers NT-proBNP and cardiac troponin.

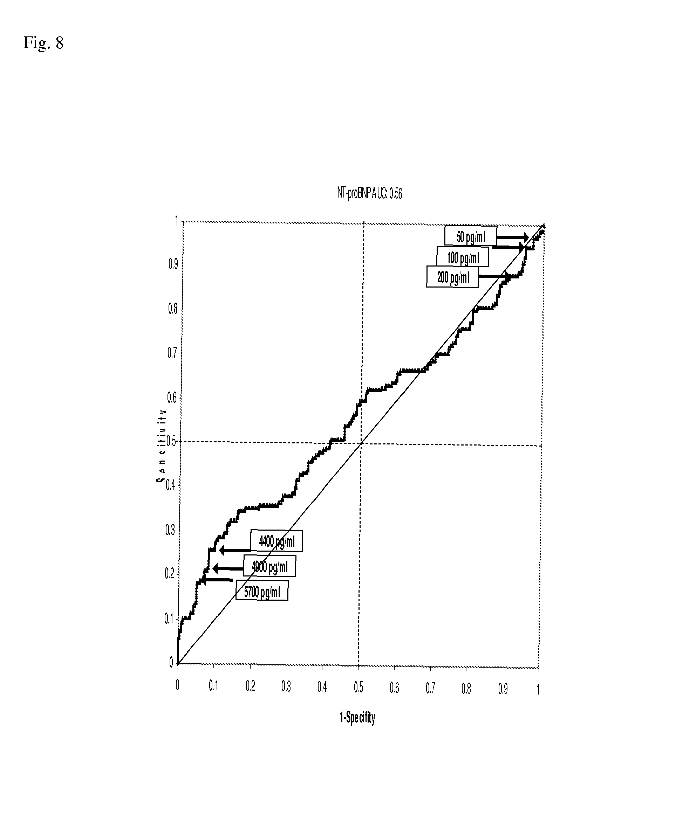

Thus, as shown in Figure 8 for NT-proBNP, optimal sensitivity yields, preferably, threshold amounts to be applicable as reference amounts being indicative for a subject being not at increased risk for mortality are within the range of about 50 pg/ml to about 200 pg/ml, and preferably about 100 pg/ml. Optimal specificity yields threshold amounts to be applicable as reference amounts being indicative for a subject being at increased risk for mortality are within the range of about 4,400 pg/ml to about 5,700 pg/ml, and preferably about 4,900 pg/ml. Alternatively, said threshold for NT-proBNP for a subject being at increased risk is median based, preferably, within the range of the 25^{th} percentile to the 75^{th} percentile, i.e. about 218.6 to about 3,650.2 pg/ml, more preferably about 240.5 to about 4,533.3 pg/ml or, most preferably, about 6,552.7 to about 37,428.0 pg/ml. More preferably, the median could be used as a threshold and an increased risk is indicated by a value of at least about 949.5 pg/ml, 1,132.0 pg/ml or 20,712.0 pg/ml for NT-proBNP.

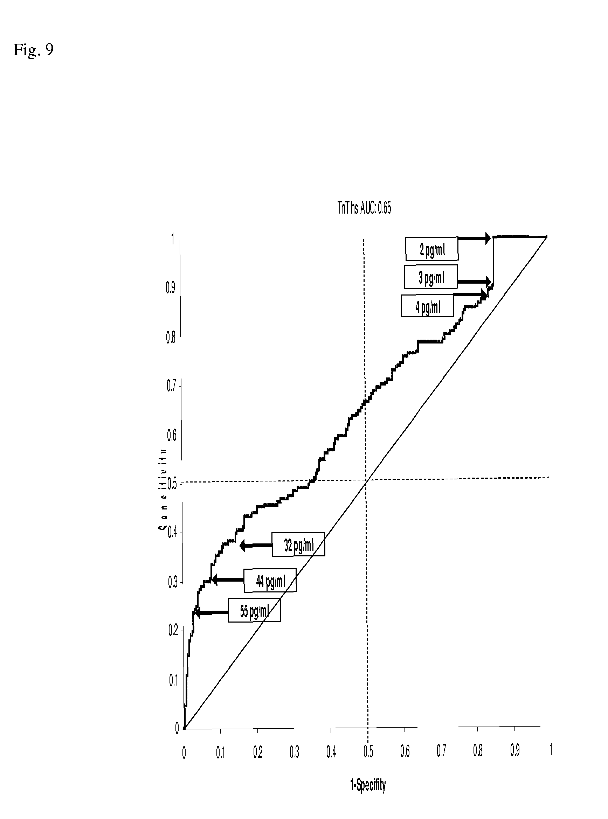

As shown in Figure 9 for troponin T, optimal sensitivity yields, preferably, threshold amounts to be applicable as reference amounts being indicative for a subject being not at increased risk for mortality are within the range of about 2 pg/ml to about 4 pg/ml, and preferably about 3 pg/ml. Optimal specificity yields threshold amounts to be applicable as reference amounts being indicative for a subject being at increased risk for mortality are within the range of about 32 pg/ml to about 55 pg/ml, and preferably about 44 pg/ml. Alternatively, said threshold for a cardiac troponin, in particular troponin T, for a subject being at increased risk is, preferably, within the range of the 25^{th} percentile to the 75^{th} percentile, i.e. about 8.48 to about 51.31 pg/ml, more preferably about 8.88 to about 54.30 pg/ml or, most preferably, about 27.37 to about 202.93 pg/ml. More preferably, the median could be used as a threshold and an increased risk is indicated by a value of at least about 17.87 pg/ml, 18.96 pg/ml or 83.09 pg/ml for troponin T

In another preferred embodiment of the method of the present invention, said method further comprises recommending an anti-sepsis therapy, if an increased risk for mortality is diagnosed.

The term "anti-sepsis therapy" as used herein refers to therapeutic measures which aim to treat or ameliorate sepsis or symptoms accompanied therewith. The term includes-drug based therapies as well as general aspects of handling of the patients, e.g., monitoring and intensive care. Such a drug based therapy may include antibiotic treatment without further delay (Gaieski 2010, Crit Care Med 38(4): 1045-1053). In addition, if not already administered, ACE inhibitors, angiotensin receptor blocker (AT 1 blockers), aldosteron- antagonists are further indicated (Saldago 2009, Expert Opinion Ther. Targets 14(1):11-20, Leone 2010, Expert Opin Emerging Drugs 15(1):1-12). In addition treatment with activated protein C requires consideration (Toussaint 2009, NEJM 361:2646-2652). Preferably, anti-sepsis therapy as used herein comprises administration of antibiotics, cortisone, hydrocortisone or complement proteins including activated protein C (commercially available under the tradename Xigris^{®}) and, preferably, ACE inhibitors, AT 1 blockers, and/or aldosternon antagonists, in addition.

The invention also discloses a method as described above wherein Transforming Growth Factor beta 1 (TGFß-1) is used instead of GDF-15.

The present invention also relates to a method for monitoring the development of acute inflammation in a subject suffering therefrom comprising:
a) determining the amount of the biomarker GDF-15 in a first and a second sample of said subject wherein said first sample has been obtained prior to said second sample; and
b) comparing the amount of GDF-15 in said first and said second sample wherein an increase in the GDF-15 amount is indicative for a diagnosis of progression of SIRS, a decrease in the amount of GDF-15 is indicative for the diagnosis of an amelioration of acute inflammation and an essentially identical amount is indicative for stagnation of acute inflammation.

The term "monitoring" as referred to above relates to keeping track of the status of the disease, i.e. acute inflammation. Monitoring includes comparing the status of the disease as reflected by the amount of the biomarker in a first sample taken at a first time point to the status of the disease reflected by the amount of the biomarker in a second sample taken at a second time point. The status of the disease may become worse and, thus, there will be progression of the disease, if the amount of the biomarker increases whereas there is amelioration and, thus, improvement of the status of the disease if the biomarker decreases. If no change is observed, i.e. an essentially identical amount is determined in the first and the second sample, the status of the disease is unchanged and the disease, thus, is stagnating. An essentially identical amount is determined if no statistically significant change in the amount is determined between the first and the second sample. Whether the amounts are essentially identical can be determined by the skilled artisan without further ado. Preferably, a change, i.e. increase or decrease is statistically significant if the amounts differ by at least about 5%, at least about 10%, at least about 15%, at least about 20%, at least about 25% or at least about 50%. Again, it is to be understood that the aforementioned method, preferably, allows monitoring in a statistically significant portion of subjects to investigated but not necessarily in all analyzed subjects.

In a preferred embodiment of the aforementioned method, said method further comprises recommending an antisepsis therapy, if progression of acute inflammation is diagnosed.

In another preferred embodiment of the aforementioned method, the biomarker(s) NT pro-BNP and/or a cardiac troponin is/are determined in addition to GDF-15.

In general, the present invention contemplates the use of the biomarker GDF-15 or a detection agent that specifically binds thereto for diagnosing whether the subject is at increased risk for mortality based on or in a sample of the said subject suffering from acute inflammation. Thus, GDF-15 or a detection agent which specifically binds thereto, in principle, can be applied for use in diagnosing whether a subject suffering from acute inflammation is at increased risk for mortality. Moreover, included is also the use of a combination of GDF-15 and NT-proBNP and/or a cardiac troponin or a combination of detection agents therefor for diagnosing whether the subject is at increased risk for mortality based on or in a sample of the said subject suffering from acute inflammation. Thus, GDF-15 and NT-proBNP and/or a cardiac troponin or a combination of detection agents therefor, in principle, can be applied for use in diagnosing whether a subject suffering from acute inflammation is at increased risk for mortality.

The term "detection agent" as used herein refers to an agent which is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or ap-tamere which specifically binds to the biomarker.

The present invention also pertains to a device adapted for carrying out the methods of the present invention disclosed above comprising:
a) an analyzing unit comprising a detection agent which specifically binds to GDF-15 adapted for determining the amount of GDF-15 and, preferably, NT pro-BNP and/or a cardiac troponin in a sample of a subject; and
b) an evaluation unit for comparing the determined amount with a reference whereby it can be diagnosed whether a subject is at increased risk for mortality or with a second amount whereby progression or amelioration of acute inflammation can be diagnosed, said unit comprising a database with (i) reference values derived from a subject suffering from acute inflammation known to be at increased risk for mortality or subjects known to be not at said increased risk for mortality or (ii) values of the amount of GDF-15 from a second sample and a computer-implemented algorithm for carrying out a comparison.

The term "device" as used herein relates to a system comprising the aforementioned units operatively linked to each other as to allow the diagnosis or monitoring according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references are either derived from a subject suffering from acute inflammation known to be at increased risk for mortality or subjects known to be not at said increased risk for mortality or values of the amount of the biomarker(s) from a second sample as described elsewhere herein. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician..

Moreover, the present invention encompasses a kit adapted for carrying out the above disclosed methods of the present invention comprising a detection agent for the biomarker(s) GDF-15 and, preferably, for NT proBNP and/or a cardiac troponine as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device.

All references cited in this specification are herewith incorporated by reference with respect to their entire disclosure content and the disclosure content specifically mentioned in this specification.

### FIGURES

Figure 1 shows a association between sensitive Troponin T and the APACHE Score.
Figure 2 shows an association between NT-proBNP and the APACHE II Score.
Figure 3 shows an association between GDF-15 and the APACHE II Score.
Figure 4 shows an association between sensitive Troponin T and the SOFA Score.
Figure 5 shows an association between GDF-15 and the SOFA Score.
Figure 6 shows an association between NT-pro BNP and the SOFA Score.
Figure 7 shows a ROC plot analysis for GDF-15, wherein rule out (optimal sensitivity) and rule in (optimal specificity) thresholds are indicated.
Figure 8 shows a ROC plot analysis for NT-proBNP, wherein rule out (optimal sensitivity) and rule in (optimal specificity) thresholds are indicated.
Figure 9 shows a ROC plot analysis for troponin T, wherein rule out (optimal sensitivity) and rule in (optimal specificity) thresholds are indicated.

### EXAMPLES

### Example 1: Determination of biomarkers GDF-15, NT-proBNP and sensitive troponine T in emergency patients

GDF-15 and NT-proBNP were determined with sandwich immuno-assays using COBAS-analyzers from Roche/Hitachi. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. The measuring range of the GDF-15 assay is 300 pg to 20000 pg. NT-proBNP amounts between 2 pg/ml and 35000 pg/ml can be measured.

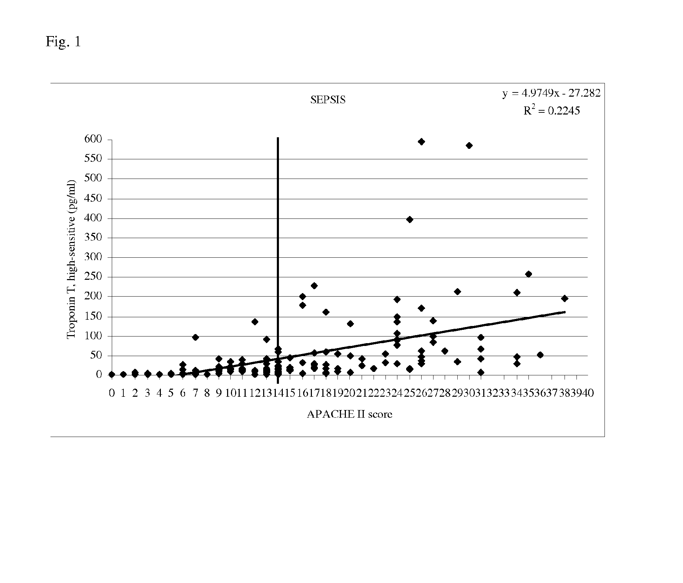

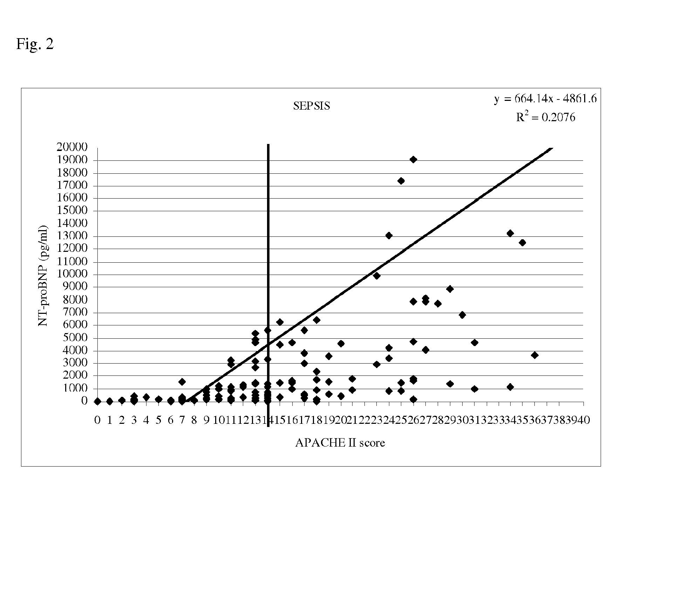

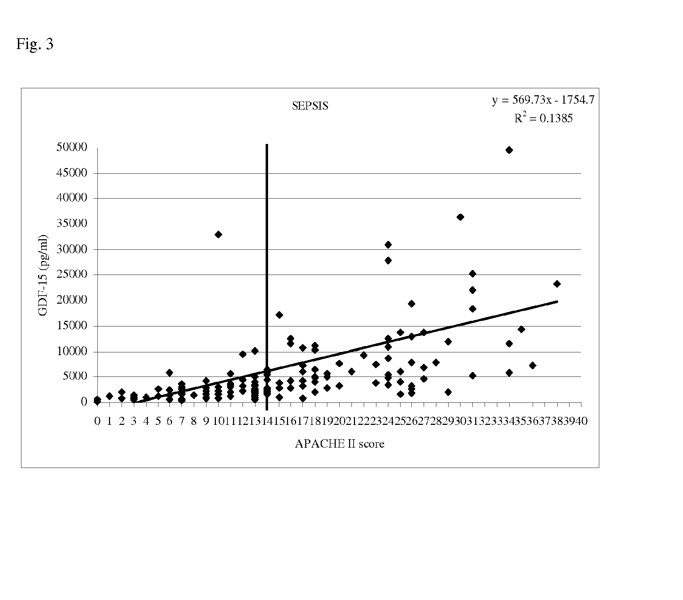

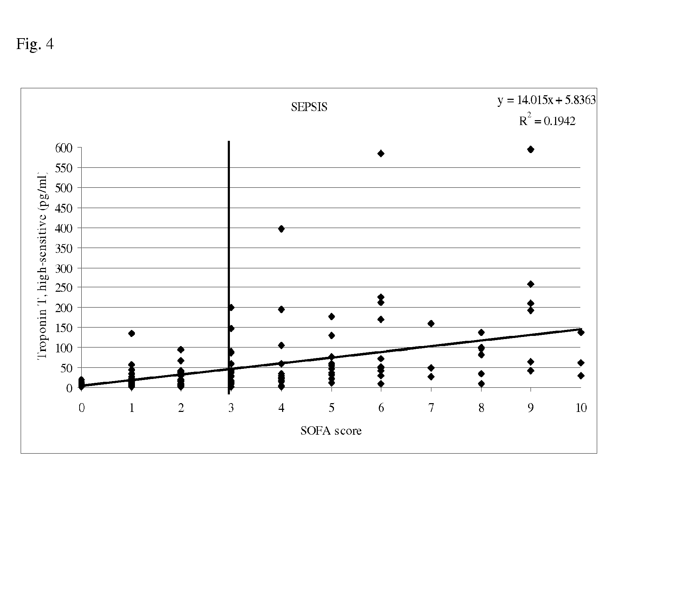

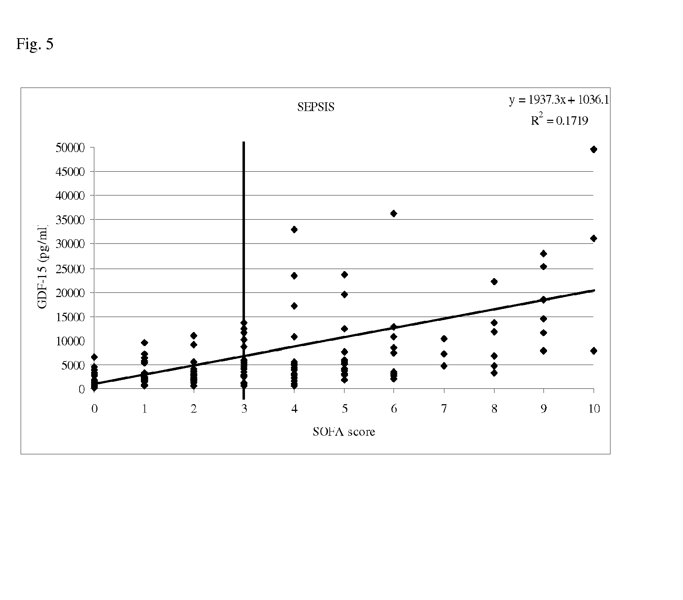

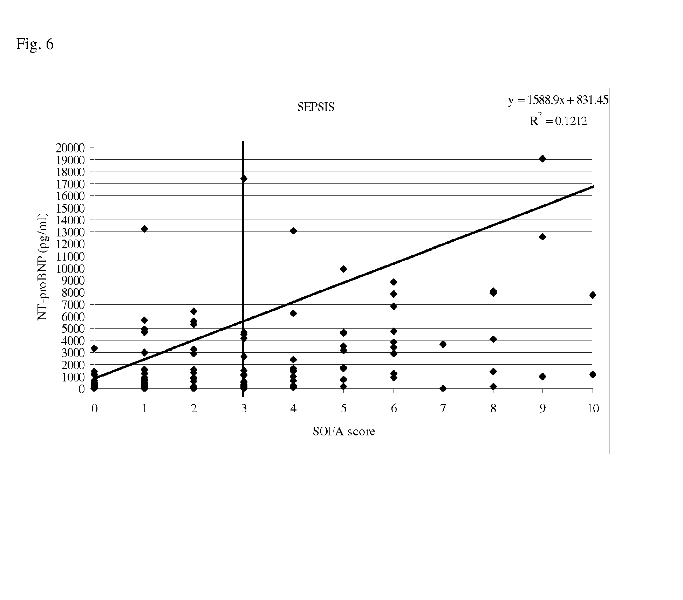

The present study contained 212 patients presenting to the emergency department with a body temperature exceeding 38.5°C, a heart rate above 90 beats per minute and a ventilatory frequency above 20 bpm. Patients on dialysis or with impaired kidney function were excluded from the study, at presentation in none of the patients acute kidney injury was diagnosed. They were 102 females (mean age 65 years (range 21 to 91 years) and 110 males, mean age 62 years (range 18 to 94 years)) In all patients the APACHE II Score and the SOFA Score were recorded, in addition death at 30 days. In the population studied median APACHE II Score was 14 (range 0 - 38) und median SOFA Score was 3 (range 0 - 10), this is also shown in Figures 1 to 6. Patients were followed for up to 3 days after presentation and blood samples were drawn 6, 24 and 72 hours after presentation.

A control group consisted of 239 patients with symptomatic but stable heart failure, all patients had a kidney function within the normal range as measured by creatinine levels within normal. They were 186 males and 53 females, mean age 61.2 years, ischemic heart disease was present in 212 patients, all others had non-ischemic heart disease.

In addition 149 clinically healthy individuals were included into the study, they had repeatedly normal blood pressure, a normal electrocardiogram, no diabetes and no history of cardiac disease or other disorders that would have put the patient at increased risk of cardiac disorders.

NT-proBNP, sensitive Troponin T and GDF-15 were measured in all patients as previously described. In Table 1, the 25th, 50th and 75th percent percentiles for GDF-15, sensitive Troponin T and NT-proBNP are given for the different groups of individuals/patients tested. Increased GDF-15, NT-proBNP and sens Troponin T levels are indicative for infectious disease complications. Severity of complications by infection increased with increasing levels of GDF-15, sens Troponin T and NT-proBNP.

**Table 1:**

| **GDF-15 pg/ml** | | | | | |
|---|---|---|---|---|---|
| **Percentile** | **Healthy Individuals** | **Heart Failure** | **Acute Inflammation, total** | **Acute Inflammation, Survivors** | **Acute Inflammation, lethal** |
| **N** | 149 | 239 | 212 | 199 | 13 |
| **25th perc.** | 503 | 1032.5 | 1980.1 | 1870.4 | 5896.9 |
| **50th perc. (median)** | 580 | 1593.8 | 3652.5 | 3421.8 | 11518 |
| **75th perc.** | 683.5 | 2738.7 | 7623.1 | 7161.2 | 22708 |
| | | | | | |

| | | | | | |
|---|---|---|---|---|---|
| **proBNP pg/ml** | | | | | |
| **Percentile** | **Healthy Individuals** | **Heart Failure** | **Acute Inflammation, total** | **Acute Inflammation, Survivors** | **AcuteInflammation, lethal** |
| **N** | 149 | 239 | 212 | 199 | 13 |
| **25th perc.** | 18.45 | 285.7 | 240.5 | 218.6 | 6552.7 |
| **50th perc. (median)** | 37.25 | 730.7 | 1132.0 | 949.5 | 20712 |
| **75th perc.** | 67.58 | 2028.0 | 4533.3 | 3650.2 | 37428 |
| | | | | | |

| **Troponin T hs pg/ml** | | | | | |
|---|---|---|---|---|---|
| **Percentile** | **Healthy Individuals** | **Heart Failure** | **Acute Inflammation, total** | **Acute Inflammation, Survivors** | **Acute Inflammation, lethal** |
| **N** | 149 | 239 | 212 | 199 | 13 |
| **25th perc.** | 0 | 6.1 | 8.88 | 8.48 | 27.37 |
| **50th perc. (median)** | 0 | 12.1 | 18.96 | 17.87 | 83.09 |
| **75th perc.** | 0 | 23.9 | 54.30 | 51.31 | 202.93 |
| | | | | | |

NT-proBNP, sensitive Troponin T and GDF-15 were correlated to the APACHE II and the SOFA Scores. As can be seen from Fig 1 - 6, levels of NT-pro BNP, sensitive Troponin T and GDF-15 correlated with the APACHE 2 and SOFA score, however all markers added significantly to these Scores, as cardiac involvement (as measured by NT-proBNP and sensitive Troponin T) was significantly different at the same Score (APACHE II or SOFA).(Figure 1 to 6). Thus, Troponin T, NT-proBNP and GDF-15 provide information independent from APACHE II and SOFAC Scores with focus on cardiac involvement known to be the primary cause of death in these individuals.

### Example 2: Case studies on individual patients

Detailed analysis of individual patients revealed that all patients presenting with high GDF-15, NT-proBNP and Troponin T levels died within 24 hours (see Table 2 below))

**Table 2:**

| **Patient Nr.** | **GDF 15** | **NT-pro BNP** | **Troponin T** |
|---|---|---|---|
| 7 | 100000 | 20713 | 50,7 |
| 35 | 6902 | 7882 | 83,1 |
| 48 | 4891 | 72324 | 149,0 |
| 66 | 9216 | 39788 | 18,3 |
| 100 | 23343 | 35086 | 195,5 |
| 113 | 36375 | 6838 | 586,0 |
| 119 | 11518 | 24816 | 210,0 |
| 166 | 17193 | 6266 | 14,0 |

GDF-15 in conjunction with NT-pro BNP was the best indicator for short term poor outcome. As becomes evident from Table 2, the combined determination of GDF-15, Troponin and NT-proBNP capture all patients dying within 24 hours, if a GDF-15 is measured above 10000 pg/ml, a NT-proBNP above 20000 pg/ml and a Troponin T levels above 50 pg/ml. These test results can be obtained within a time period of 15 minutes and these patients, in case they meet the above criteria are subject to intense and broad antibiotic therapy, in need of immediate intensive care, surveillance and supportive therapy in this unit. In a different type of analysis patients with GDF-15 levels of more than 5170, 5940 and 6600 pg/ml and NT-pro BNP levels above 4400, 4900, 5700 pg/ml and Troponin T levels above 32,44 and 55 pg/ml were found to be at increased risk of mortality (Figures 7 - 9). These patients also deserve intense monitoring, early antibiotic therapy, but may not be candidates for immediate intensive care unit surveillance. The same analysis also provided evidence of low risk patients, who are unlikely to benefit from antibiotic therapy and who are candidates for early discharge, they have GDF-15 values below 770, 660 and 550 pg/ml, NT-pro BNP levels below 200, 100 and 50 pg/ml and Troponin T levels below 4, 3 and 2 pg/ml. These values are also found in healthy subjects or patients with mild heart failure.

In contrast in patients who survived, GDF-15 was superior to NT-pro BNP to indicate recovery. Specifically and surprisingly, GDF-15 decrease after 6 hours after presentation predicted recovery. GDF-15 was found to be highly "stable" in patients with heart failure with more fluctuation recognized for NT-pro BNP, thus a decrease of NT-pro BNP of at least 20 % and a decrease of GDF-15 of at least 10 % was considered as improvement. As GDF-15 reflects an inflammatory state and NT-pro BNP reflects cardiac function concordance of both test results reflect a similar trend in inflammation and cardiac function whereas discordant test results suggest differences in change of inflammatory and cardiac function components. A laboratory response (decrease of GDF-15 and or NT-proBNP at 6 h) was followed by a decrease of the APACHE II score of at least 3 and of the SOFA Score by at least one point at 72 h, thus suggesting an early sign (prediction of) of recovery. This becomes evident from the following cases (see Table 3 below).

**Table 3:**

| **Pat. ID** | **Time** **Point** | **Troponin T** **pg/ml** | **proBNP** **pg/ml** | **GDF-15** **pg/ml** |
|---|---|---|---|---|
| **15** | Admission | 53.8 | 2918.6 | 7473.7 |
| | 6 hours | 99.0 | 6217.6 | 10059.4 |
| | 24 hours | 67.3 | 8437.7 | 7150.86 |
| | 72 hours | | | |
| **28** | Admission | 131.8 | 4558.2 | 7772.6 |
| | 6 hours | 167.5 | 9113.6 | 14227.1 |
| | 24 hours | 131.4 | 38176.0 | 4415.77 |
| | 72 hours | | | |
| **30** | Admission | 77.7 | 775.5 | 5543.5 |
| | 6 hours | 76.9 | 612.0 | 7163.3 |
| | 24 hours | 57.7 | 1313.0 | 3679.84 |
| | 72 hours | 43.9 | 4943.4 | 3217.44 |
| **37** | Admission | 5.8 | 704.6 | 6575.8 |
| | 6 hours | | | |
| | 24 hours | 5.2 | 2693.3 | 2868.41 |
| | 72 hours | 7.7 | 10283.5 | 3881.49 |
| **60** | Admission | 43.9 | 4508.4 | 2796.2 |
| | 6 hours | 41.1 | 8534.7 | 3401.8 |
| | 24 hours | 36.5 | 6432.9 | 2023.78 |
| | 72 hours | | | |
| **64** | Admission | 35.4 | 1402.9 | 11863.4 |
| | 6 hours | 25.2 | 1743.9 | 7389.4 |
| | 24 hours | 24.7 | 1168.2 | 4460.83 |
| | 72 hours | 15.2 | 570.0 | 3833.28 |
| **114** | Admission | 62.8 | 7729.2 | 7910.8 |
| | 6 hours | 73.0 | 10534.4 | 14840.0 |
| | 24 hours | 94.5 | 17794.2 | 9288.51 |
| | 72 hours | 66.2 | 14936.8 | 5689.63 |
| **169** | Admission | 14.1 | 17403.6 | 13689.5 |
| | 6 hours | 13.2 | 22289.9 | 17519.8 |
| | 24 hours | 62.7 | 52980.3 | 15383.38 |
| | 72 hours | 27.5 | 32683.3 | 3995.75 |

In other cases improvement was recognised by a decrease of GDF-15 and NT-proBNP as shown in the following cases: (see Table 4 below)

**Table 4:**

| **Pat. ID** | **Time** **Point** | **Troponin T** **pg/ml** | **proBNP** **pg/ml** | **GDF15** **pg/ml** |
|---|---|---|---|---|
| **13** | Admission | 138.5 | 8101.0 | 13733.9 |
| | 6 hours | | | |
| | 24 hours | 133.8 | 6463.1 | 12593.17 |
| | 72 hours | 100.8 | 5018.2 | 7142.31 |
| **25** | Admission | 9.1 | 840.7 | 3090.9 |
| | 6 hours | 11.5 | 1151.9 | 3158.8 |
| | 24 hours | 67.3 | 8437.7 | 7150.86 |
| | 72 hours | | | |
| **75** | Admission | 66.1 | 30344.8 | 25230.0 |
| | 6 hours | 42.8 | 16096.9 | 12151.1 |
| | 24 hours | 53.6 | 2311.4 | 4107.79 |
| | 72 hours | 39.5 | 1823.1 | 4806.27 |
| **99** | Admission | 12.3 | 308.3 | 2288.0 |
| | 6 hours | 15.4 | 1269.5 | 2865.0 |
| | 24 hours | 8.4 | 564.1 | 1426.15 |
| | 72 hours | 3.4 | 307.8 | 1137.74 |
| **170** | Admission | < 3.0 | 1177.9 | 4478.7 |
| | 6 hours | < 3.0 | 694.0 | 2619.1 |
| | 24 hours | < 3.0 | 259.6 | 1138.91 |
| | 72 hours | 9.3 | 630.4 | 1218.81 |
| **174** | Admission | 54.3 | 3540.1 | 5109.5 |
| | 6 hours | | | |
| | 24 hours | 47.0 | 3144.4 | 2313.48 |
| | 72 hours | 50.4 | 2020.2 | 3251.25 |

These examples show that NT-pro BNP und preferentially GDF-15 can be used to monitor reversal of the infection

## Claims

1. A method for diagnosing whether a subject suffering from acute inflammation is at increased risk for mortality comprising
a) determining the amount of the biomarker GDF-15 in a sample of said subject; and
b) comparing said amount to a reference amount whereby an increased risk for mortality is to be diagnosed.

2. The method of claim 1, wherein the amount of the biomarker(s) NT pro-BNP and/or a cardiac troponin is determined in addition to GDF-15.

3. The method of claim 1 or 2, wherein said reference amount is derived from a subject or a group of subjects suffering from acute inflammation known to be at increased risk for mortality.

4. The method of claim 3, wherein an essentially identical or increased amount for the biomarker(s) in the sample compared to the reference amount is indicative for a subject being at increased risk for mortality and wherein a decreased amount for the biomarker(s) in the sample compared to the reference is indicative for a subject being not at increased risk for mortality.

5. The method of claim 1 or 2, wherein said reference amount is derived from a subject or group of subjects suffering from acute inflammation known to be not at increased risk for mortality.

6. The method of claim 5, wherein an essentially identical or decreased amount for the biomarker(s) in a sample compared to the reference amount is indicative for a subject being not at increased risk for mortality and wherein an increased amount for the biomarker(s) in the sample compared to the reference is indicative for a subject being at increased risk for mortality.

7. The method of any one of claims 1 to 6 wherein said method further comprises recommending an anti-sepsis therapy, if an increased risk for mortality is diagnosed.

8. A method for monitoring the development of acute inflammation in a subject suffering therefrom comprising:
a) determining the amount of the biomarker GDF-15 in a first and a second sample of said subject wherein said first sample has been obtained prior to said second sample; and
b) comparing the amount of GDF-15 in said first and said second sample wherein an increase in the GDF-15 amount is indicative for a diagnosis of progression of acute inflammation, a decrease in the amount of GDF-15 is indicative for the diagnosis of an amelioration of acute inflammation and an essentially identical amount is indicative for stagnation of acute inflammation.

9. The method of claim 8, wherein said method further comprises recommending an anti-sepsis therapy, if progression of acute inflammation is diagnosed.

10. The method of claim 8 or 9, wherein the biomarker(s) NT pro-BNP and/or a cardiac troponin is determined in addition to GDF-15.

11. The method of claim 7, 9 or 10 wherein said antisepsis therapy comprises administration of antibiotics, cortisone, hydrocortisone or complement proteins including activated protein C.

12. The method of any one of claims 1 to 11, wherein TGFß-1 is determined instead of GDF-15.

13. Use of the biomarker GDF-15 or a detection agent that specifically binds thereto in a sample of a subject suffering from acute inflammation for diagnosing whether the subject is at increased risk for mortality.

14. A device adapted for carrying out the method of any one of claims 1 to 12 comprising
a) an analyzing unit comprising a detection agent which specifically binds to GDF-15 adapted for determining the amount of GDF-15 and, preferably, NT pro-BNP and/or a cardiac troponin in a sample of a subject; and
b) an evaluation unit for comparing the determined amount with a reference amount whereby it can be diagnosed whether a subject is at increased risk for mortality or with a second amount whereby progression or amelioration of acute inflammation can be diagnosed, said unit comprising a database with (i) reference amount values derived from a subject as defined in claim 3 or 5 or (ii) values of the amount of GDF-15 from a second sample and a computer-implemented algorithm for carrying out a comparison.

15. A kit adapted for carrying out the method of any one of claims 1 to 12 comprising a detection agent for the biomarker(s) GDF-15 and, preferably, for NT pro-BNP and/or a cardiac troponine as well as instructions for carrying out the said method.
